# EUROPEAN PATENT SPECIFICATION

(11) **EP 1 948 679 B1**
(45) Date of publication and mention of the grant of the patent: **05.05.2010**
(21) Application number: 06799316.2
(22) Date of filing: 18.10.2006
(51) Int. Cl.: C07K 14/00, C12N 15/81, C07K 14/47

(54) **METHOD FOR PREPARING RECOMBINANT PEPTIDE FROM SPIDER VENOM AND ANALGESIC COMPOSITION CONTAINING THE PEPTIDE**
VERFAHREN ZUR HERSTELLUNG EINES REKOMBINANTEN PEPTIDS AUS SPINNENGIFT, UND DAS PEPTID ENTHALTENDE ANALGETISCHE ZUSAMMENSETZUNG
METHODE DE PREPARATION D'UN PEPTIDE DE RECOMBINAISON A PARTIR DE VENIN D'ARAIGNEE ET COMPOSITION ANALGESIQUE CONTENANT CE PEPTIDE

(30) Priority: 18.10.2005 KR 20050098082; 06.01.2006 KR 20060001918
(43) Date of publication of application: 30.07.2008
(73) Proprietor: Seoul National University Industry Foundation, Gwanak-ku Seoul 151-050 (KR)
(72) Inventor: OH, Uhtaek, Sungnam-si 463-050 (KR); KIM, Byung Moon, Seoul 135-270 (KR); PARK, Seung Pyo, Seoul 151-872 (KR); NA, Heung Sik, Seoul 137-775 (KR)
(74) Representative: Wibbelmann, Jobst
(86) International application number: PCT/KR2006/004242
(87) International publication number: WO 2007/046634

(56) References cited:
- EP-A2- 0 707 067
- PARK S P ET AL: "A tarantula spider toxin, GsMTx4, reduces mechanical and neuropathic pain" PAIN, ELSEVIER SCIENCE PUBLISHERS, AMSTERDAM, NL, vol. 137, no. 1, 30 June 2008 (2008-06-30), pages 208-217, XP025574189 ISSN: 0304-3959 [retrieved on 2008-03-24]
- DATABASE GENPEPT [Online] SUCHYNA T.M. ET AL.: 'Identification of a peptide toxin from Grammostola spatulata spider venom that blocks cation-selective stretch-activated channels', XP003012098 Retrieved from NCBI Database accession no. (1TYK_A) & J. GEN. PHYSIOL. vol. 115, no. 5, 2000, pages 583 - 598
- SUCHYNA T.M. ET AL.: 'Bilayer-dependent inhibition of mechanosensitive channels by neuroactive peptide enantiomers' NATURE vol. 430, 08 July 2004, pages 235 - 240, XP001203671
- OSTROW K.L. ET AL.: 'cDNA sequence and in vitro folding of GsMTx4, a specific peptide inhibitor of mechanisensitive channels' TOXICON. vol. 42, no. 3, September 2003, pages 263 - 274, XP002375376

## Description

### FIELD OF THE INVENTION

The present invention relates to a method for producing recombinant spider venom peptides and analgesic compositions containing the same. More specifically, the GsMTx4 gene was ligated to the signal sequence of the alpha factor (α-factor) and placed under the control of the alcohol oxidase (AOX) promoter, a methanol-inducible promoter, to construct a recombinant plasmid for yeast expression. The present invention also relates to a method for producing GsMTx4 peptides by transforming yeast with said plasmid and analgesic compositions containing said peptides.

### BACKGROUND OF THE INVENTION

Ion channels that are gated in response to mechanical stress are collectively termed as mechanosensitive ion channels (MSCs). Since most of them open up when the cell membrane is stretched, they are also referred to as stretch-activated channels (SACs) (Sachs, F., Morris, C. E., Rev. Physiol. Biochem. Pharmacol. 132, pp1∼77, 1998). In general, mechanosensitive ion channels are present in virtually all cells of the body, and are known, albeit without much active research into field, to be involved in the senses of touch and hearing, muscle contraction, blood pressure control and cell volume control (Hamill, O. P., Martinac, B., Physiol. Rev. 81, pp685∼740, 2001). It is conventional to use inhibitors specific to the ion channel of interest when studying ion channels, but mechanosensitive ion channels (MSCs) lack such specific inhibitors so that their studies have been limited to nonspecific inhibitors such as gadolinium (Gd³⁺) (Caldwell, R. A. et al., Am. J. Phys. 275, ppC619∼C621, 1998) and streptomycin (Gannier, F. et al., Circ. Res. 28, pp1193∼1198, 1994). Recently, however, a specific peptide inhibitor has been isolated from a Chilean tarantula (Sachs, F. et al., J. Gen. Physiol. 115, pp583∼598, 2000).

The specific inhibitor peptide mentioned above consists of total 34 amino acids having a molecular weight of approximately 4,094 Da with 6 cysteine residues forming 3 disulfide bonds. It was named GsMTx4, combining "Gs" for the scientific name of the tarantula spider, *Grammostola spatulata*, "M" for "mechanosensitive", "Tx" for "toxin" and 4 for the fourth peak in the active sequence (Sachs, F. et al., J. Gen. Physiol. 115, pp583∼598, 2000). Its amino acid sequence is as follows:

With such specific inhibitor of MSCs now at hand, there are reports suggesting clinical applications of this inhibitor in areas involving cellular mechanics. For instance, GsMTx4 was shown to prevent cell swelling associated with congestive heart failure, especially in the lungs, liver, bowels and legs (Sachs, F. et al., J. Gen. Physiol. 115, pp583∼598, 2000). GsMTx4 was also reported to relieve symptoms of atrial fibrillation by inhibiting stretch-activated channels (SACs) when it was administered to the hearts of rabbits after atrial fibrillation was induced with high level stress (Sachs, F. et al., Nature 409, pp35∼36, 2001; Hamill, O. P., Martinac, B., Physiol. Rev. 81, pp685∼740, 2001). This suggests a use of GsMTx4 as a cure for heart diseases in that it is able to hold back atrial fibrillation, a main cause of heart failure. In addition, GsMTx4-mediated blocking of SACs may be highly effective in treating brain tumors, since an invasion of tumor into the brain tissue entails abnormal changes in the surrounding cells as to secrete tumor-promoting growth factors through SACs (Sachs, F. et al., J. Gen. Physiol. 115, pp583∼598, 2000).

While carrying out studies on toxins that block ion channels, the present inventors took notice of this spider toxin peptide specific to MSCs, and conceived this invention assuming that such specific inhibition of MSCs might extend to suppressing pains as well. The peptide mentioned above from the Chilean tarantula, however, was not readily available, and even if it had been so, the amount one could isolate from the spiders were limited to trace amounts; thus, there was a need for a stable production method of this inhibitor peptide.

The GsMTx4 peptide, as noted above, has 6 cysteine residues forming a cysteine knot structure characterized as the ICK motif (Oswald, R. E. et al., J. Biol. Chem. 277(37):34443∼34450, 2002). Such structural uniqueness restricts its production by routine chemical synthesis. One may resort to genetic engineering methods besides chemical synthesis for producing useful proteins of limited availability using such various expression systems as bacteria, yeasts, fungi, plants and animals. Protein synthesis through *Escherichia coli* (*E. coli*), however, may require additional operations when producing peptides from higher organisms since the bacterium lacks mechanisms for glycosylation or proper secretion outside the cell. Such complications make the goal of attaining the native 3-dimensional structure of the protein quite problematic. Therefore, to obtain a peptide that maintains the native 3-dimensional structure of GsMTx4, it is more advantageous to employ extracellular secretion routes. Unlike *E. coli,* yeasts, despite being microbes, can secrete proteins to the extracellular medium and are equipped with the machinery for controlling eukaryotic expression and cell division, and modifying secreted proteins; thus they are highly useful for producing recombinant proteins from higher eukaryotes (Marten & Seo, Chap. 7, Expression systems and processes for rDNA products, eds. Hatch et al., ACS Symp. ser. 477, 1991). Accordingly, the present inventors attempted a production of recombinant spider toxin peptides using a yeast expression system. So far, there have not been published studies reporting GsMTx4 production in yeast expression systems.

Under this rationale, the present inventors focused on the direction of yeast expression and have succeeded in constructing a recombinant plasmid carrying the gene for the spider toxin peptide and developing a stable production method for the same using yeast transformed with said plasmid. The present inventors also have confirmed that the toxin peptide obtained from the above mentioned method inhibits the activation by mechanical stress of mechanosensitive ion channels present on the surface of nociceptive neurons, thereby completing the invention.

### DETAILED DESCRIPTION OF THE INVENTION

In the following, the present invention is described in detail.

The present invention provides a recombinant plasmid for yeast expression that carries the gene for GsMTx4, a spider toxin peptide. The plasmid of the present invention contains a promoter for methanol-assimilating yeasts, signal sequence for secretion and the gene for GsMTx4. Preferably the plasmid has the alcohol oxidase (AOX) promoter, secretion signal sequence from the alpha factor SEQ. ID NO 7) and for a structural gene, the gene encoding GsMTx4. The gene for GsMTx4 is under the control of the AOX promoter and α-factor signal sequence.

Based on the published amino acid sequence (SEQ. ID NO 1), the DNA sequence encoding GsMTx4 of this invention (SEQ. ID NO 2) was designed to conform to the preferred codon usage in yeast and by taking the stability of the full-length base sequence into consideration. For instance, the codons encoding glycine, the first amino acid residue, are GGT, GGC, GGA and GGG. Out of these 4 codons, we chose GGT in consideration of typical codon usage in yeast. (Graham, S. et al., Protein Expr. Purif. 26, pp96∼105, 2002). This sequence is given below:

In the construction of the full-length nucleotide sequence (SEQ. ID NO 2) encoding GsMTx4, the whole sequence can be assembled by annealing a suitable number of overlapping, synthetic oligonucleotides forming two complementary strands. In the preferred experimental example described in this patent application, a total of 4 oligonucleotides (2 sense oligonucleotides, 2 antisense oligonucleotides) were synthesized and assembled. Another way of preparing the full-length DNA sequence would be to use polymerase chain reaction (PCR). Any combination of conventional methods available to those skilled in the art, however, may be employed for preparing this oligonucleotide sequence without being limited to those described in the experimental examples' section.

Meanwhile, the plasmid of the present invention further comprises a terminator sequence and a selection marker gene, preferably the AOX terminator and zeocin-resistance gene. This plasmid can be introduced in methanol-assimilating yeast strains for producing GsMTx4.

The AOX promoter mentioned above represses gene expression in the presence of non-methanol carbon sources and is inducible by an addition of methanol; thus it is able to fine-tune the expression of downstream genes. The AOX terminator enhances the stability of mRNA transcripts by mRNA processing such as polyadenylation. The promoter and terminator used in the inventive plasmid, however, need not be limited to the AOX promoter and terminator and may be replaced by other promoters and terminators in accordance with the objectives of this invention.

The secretion signal sequence for the α-factor is capable of effectively secreting most exogenous proteins from the host strain. This signal sequence is particularly suitable for proteins of low molecular weight. This signal sequence, however, may be replaced with a suitable secretion signal sequence in accordance with the objectives of the invention.

The yeast strain for expressing GsMTx4 carried by the plasmid of this invention is preferably a methanol-assimilating strain from the order *Pichia*, and more preferably *Pichia pastoris*. Expression in *Pichia* yeasts takes place in eukaryotic cells to support proper post-translational modification, enabling production of GsMTx4 peptides effective in terms of function and activity. In addition, the exogene fragment under the control of the AOX promoter is expected to integrate readily into the locus for the alcohol oxidase (AOX) gene native to the yeast strain. The expression of this integrated exogene can be precisely controlled with methanol. In particular, *Pichia pastoris* enjoy strong advantages in the aspects of fermentation engineering in that it allows production to be scaled up to more than 10,000 fold, and it supports expression yields far higher than those of the yeast *Saccharomyces cerevisiae*, the conventional expression system of choice. Furthermore, *Pichia pastoris* can not only express exogenous proteins in large amounts, but it also lacks α-1,3-mannosyltransferase activity so that it produces far fewer proteins with α-1,3-mannose residues, which are harmful to humans, than *Saccharomyces cerevisiae* does.

Thanks to such desirable characteristics, Pichia yeasts have been employed in the production of many exogenous proteins (Sreekrishna, K. et al., Biochemistry 28, pp4117∼4125, 1989; Clare, J. et al., Bio/Technology 9, pp455∼461, 1991; Ikegaya, K. et al., Anal. Chem. 69, pp1986∼1991, 1997); however, no studies on expressing GsMTx4, a Chilean tarantula toxin peptide, in this yeast have been reported up to date.

As a preferred embodiment in the experimental examples' section, the present inventors provide pGSMTX4, a recombinant expression plasmid for GsMTx4 in which the GsMTx4 gene is introduced in the vector pPICZαB.

We noted pPICZαB as a suitable vector for constructing recombinant expression plasmids for GsMTx4 to be used in methanol-assimilating yeasts. The pPICZαB contains the AOX promoter, secretion signal sequence from the α-factor, AOX terminator, and zeocin-resistance gene as a selection marker. The AOX promoter represses gene expression in the presence of non-methanol carbon sources and is inducible by an addition of methanol, enabling a finely tuned expression of downstream genes. This promoter also supports a high level expression of exogenous proteins in Pichia yeasts. The AOX terminator enhances the stability of mRNA transcripts by mRNA processing such as polyadenylation. The secretion signal sequence for the α-factor is capable of effectively secreting most exogenous proteins from the host strain. This signal sequence consists of 85 amino acids starting with the ATG initiator codon. This sequence is linked at its C-terminus, Glu-Lys-Arg, which corresponds to the 83, 84, and 85^{th} residue, respectively, to the N-terminal glycine codon GGT of GsMTx4 via a Glu-Ala-Glu-Als sequence in between. Since the Kex2 protease, which recognizes the Glu-Lys-Arg sequence, is present in the *trans*-Golgi network of yeasts, the synthesized polypeptide ppL::GsMTx4 becomes processed in the *trans*-Golgi network and the signal sequence is cleaved. On the other hand, the Glu-Ala-Glu-Ala sequence is cleaved upon secretion by the Ste13 protease which recognizes this sequence. Therefore, the final form of the GsMTx4 peptided secreted to the medium is an intact, full-length GsMTx4 with its N-terminus beginning with glycine.

In another aspect of the invention, the present inventors provide a method for producing the spider toxin peptide GsMTx4 using yeast transformed with the recombinant plasmid of this invention. The production method of GsMTx4 using a methanol-assimilating yeast comprises the steps of:
1) transforming methanol-assimilating yeast with the recombinant plasmid of the present invention;
2) inducing the expression of GsMTx4 by adding methanol to the culture medium of the methanol-assimilating yeast cells of step 1)
3) collecting GsMTx4 from the culture medium of step 2).

The induction of expression in step 2) involves adding methanol to a content less than 5%, or preferably less than 0.5% to the culture medium to induce the expression of GsMTx4 via the AOX promoter by culturing for less than 10 days, or preferably 5 days.

In a preferred embodiment of the invention, the gene sequence for GsMTx4 was adapted from its published sequence in order to raise the stability of the DNA sequence and conform to the conventional codon usage in yeast. Accordingly, 4 synthetic oligonucleotides with engineered restriction sites were assembled, amplified to produce the adapted gene sequence and it was subcloned into a backbone vector. The present inventors used pPICZαB (Invitrogen, USA) as the backbone vector to construct the final vector for overexpressing GsMTx4. pPICZαB contains a zeocin-resistance gene as a selection marker, and the AOX promoter, which achieves far stronger expression than the conventional GAL promoters, and is capable of induced expression using methanol as the carbon source. The amplified GsMTx4 gene was subcloned at the *Xho*I/*Xba*I position of pPICZαB to construct the expression plasmid pGSMTX4. DH5α *E. coli* cells were then transformed with pGSMTX4. The present inventors deposited the *E. coli* transformants carrying the gene for GsMTx4 at the Korea Culture Center of Microorganisms (KCCM, 361-221 Hongje-dong, Seoul, Korea) on April 9, 2006 with the accession number KCCM10652p.

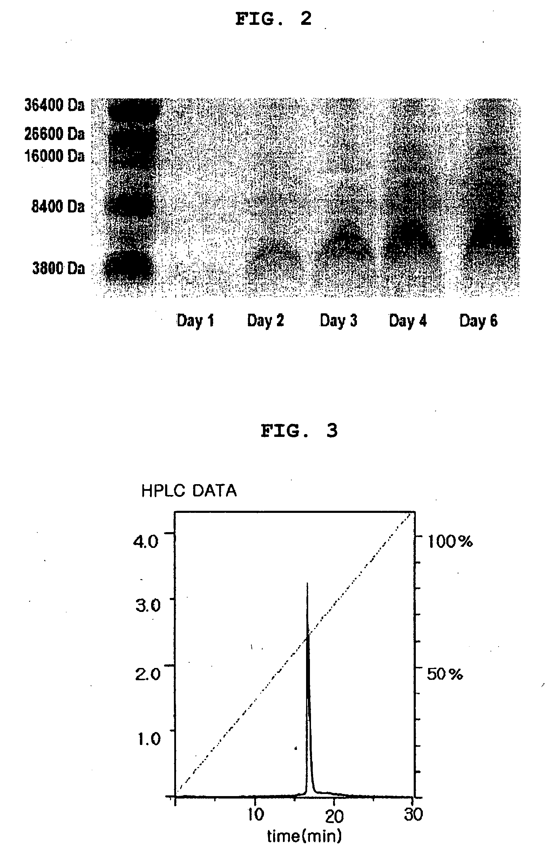

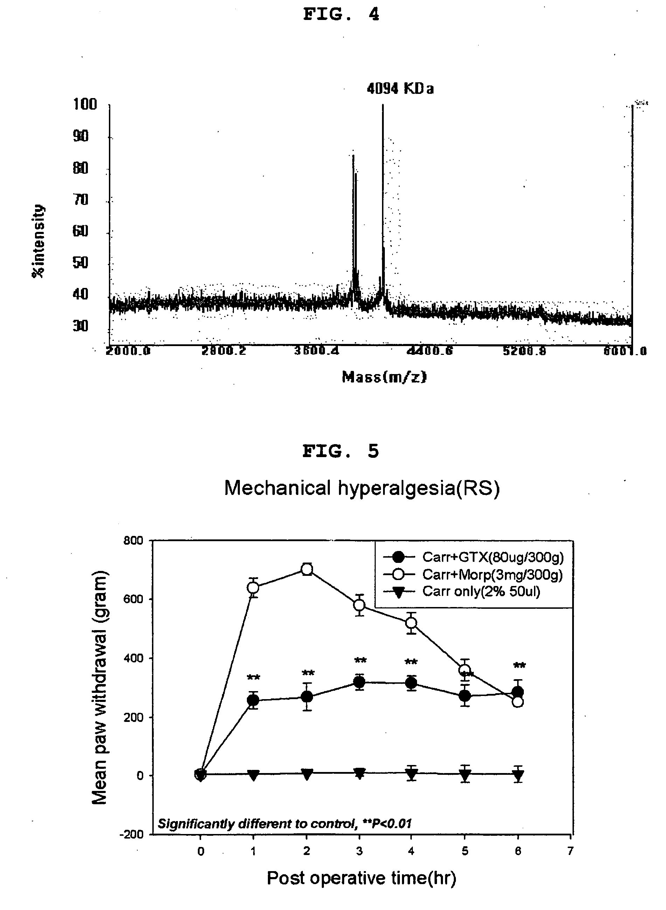

The recombinant expression plasmid mentioned above was amplified inside *E. coli* cells and isolated. It was linearized by a restriction digest and introduced at the genomic alcohol oxidase (AOX) gene locus by electroporation. In a preferred embodiment of the invention, the present inventors chose *Pichia pastoris* GS115 (his4, His⁻Mut⁺) (Invitrogen, USA) among the *Pichia* yeast to be transformed with the linearized plasmid. Later, colonies were picked from the surface of the culture medium containing an antibiotic and tested for the presence of the GsMTx4 gene by PCR. The selected transformants were then inoculated in minimal media, followed by an addition of methanol to activate the genomic AOX promoter, which in turn induces transcription of the downstream GsMTx4 gene, leading to the production of GsMTx4. When the culture medium was collected and analyzed by high performance liquid chromatography, a single peak of protein eluted (See FIG. 3). The final yield of the GsMTx4 peptide was estimated using a spectrophotometer to be approximately 100 mg per liter. An additional matrix-assisted laser desorption/ionization time-of-flight (MALDI-TOF) mass spectrometry analysis confirmed that the prepared peptide had a molecular weight of 4,094 Da (FIG. 4), in agreement with the value anticipated from the DNA sequence.

In still another aspect of this invention, the present inventors provide analgesic compositions containing the GsMTx4 peptide or its derivatives as the main ingredient.

In order to test whether the GsMTx4 peptide of this invention and its derivatives (Korean patent application number 2005-0098082) have the same amino acid sequence as published in the literature (Oswald, R. E. et al., J. Biol. Chem. 277(37), pp34443∼34450, 2002), the present inventors analyzed their molecular weights by MALDI-TOF mass spectrometry and confirmed their sequences with Edman degradation. They had the same amino acid sequence as SEQ. ID NO 1.

Mechanosensitive ion channels are gated by such stimuli as mechanical stress and temperature (e.g. high or low temperatures). It is known that the GsMTx4 peptide inhibits these ion channels to suppress volume-activated currents, which in turn leads to a 4blocked pain signaling. In order to test the analgesic efficacies of GsMTx4 and its derivatives, the present inventors carried a battery of tests as described below. In these experiments, pain inducers, mechanical stress, high or low temperatures may be used as the external stimulus. As for pain inducers, carrageenan, formalin and lysophosphatidic acid (LPA) may be used. For an induction of hyperalgesia, a secondary effect of inflammatory pain, carrageenan is particularly preferred.

The present inventors carried out the experiments described below with mice after an injection of either carrageenan or saline solution at mouse hind paws followed by an intraperitoneal injection of either GsMTx4 or, for the positive control group, morphine.

### 1. The Randall-Selitto test

In order to probe mechanical hyperalgesia in mice, the present inventors conducted the Randall-Selitto test, in which severe mechanical stress is applied to inflict pain. In detail, force is applied to a sharp-ended rod in touch with a hind paw of a mouse. As a measure of pain sensitivity, the weight of the rod at the pain threshold is recorded.

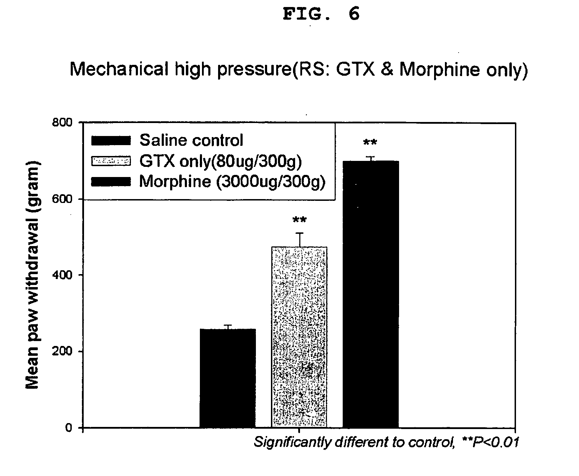

The results show that the inflammation caused by the carrageenan treatment brought down the pain threshold weight by about 50%. When these carrageenan-treated mice received an intrapertoneal injection of GsMTx4, their pain threshold weights were substantially higher, which was also the case with the central analgesic morphine for the positive control group. This indicates that as in the case of morphine, GsMTx4 has an analgesic effect (See Figures 5 and 6).

### 2. The von Frey test

In order to assess mechanical allodynia in mice, the present inventors conducted the von Frey test, in which weak stimuli or rubbing stress is applied to inflict allodynic pain. In detail, one the limbs of a mouse was injected with pain inducers such as carrageenan and formalin to incur pain. In such a case, the injected limb is sensitive to even very small mechanical stimuli. Two thin von Frey hairs with different widths were used to differentiate the pain thresholds.

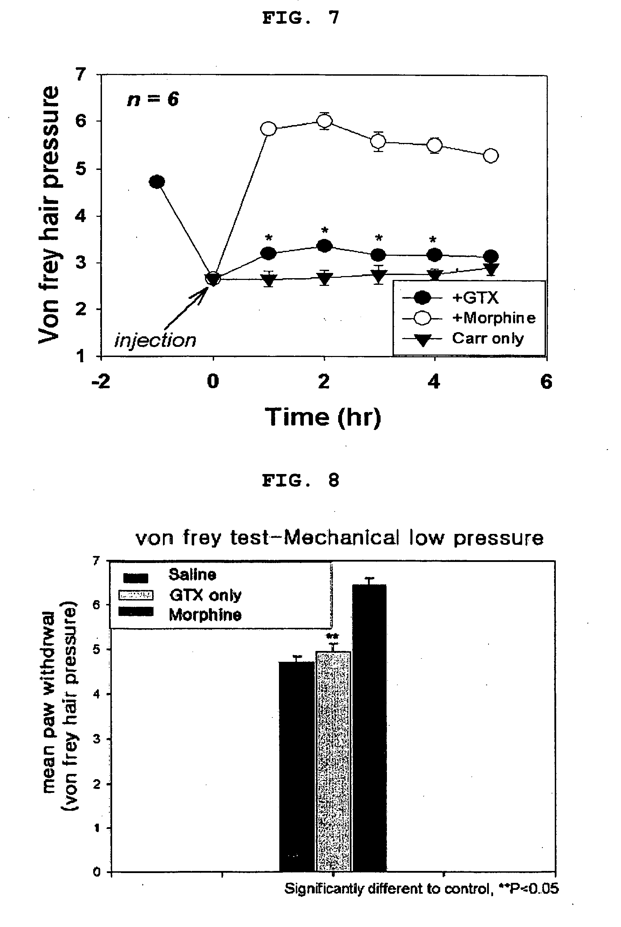

There was no difference in the endurance against hair force among mice treated with saline solution alone and those mice that did not receive saline. Mice injected with 2% carrageenan in one of their limbs showed a large drop in their pain threshold for hair force. When GsMTx4 or morphine was injected to the carrageenan-treated mice, both injections were observed to reduce hypersensitivity. This indicates that as in the case of morphine, GsMTx4 has an analgesic effect (See Figures 7 and 8).

### 3. The weight-bearing test

In order to monitor behavioral changes brought by complex mechanical stimuli, the present inventors conducted the weight-bearing test, in which complex mechanical pain is assessed as the stimulus is varied. In detail, one the hind limbs of a mouse was injected with pain inducers such as carrageenan and formalin to incur pain. In such a case, the mouse puts less weight in the injected hind limb, but puts more weight than usual in the other, normal hind limb and this difference in weight is recorded as a measure of pain.

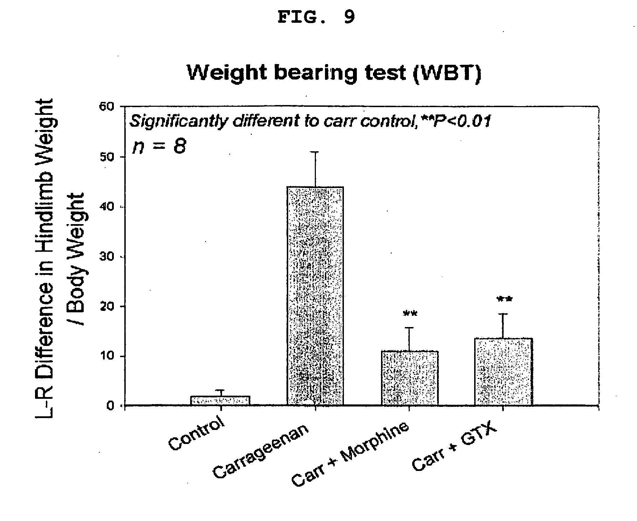

For mice treated with saline solution only, there was no weight difference between their hind limbs, but mice treated with carrageenan showed substantial weight difference between them. When GsMTx4 or morphine was injected to the carrageenan-treated mice, the substantial weight difference induced by the injection of carrageenan reverted back to a low value (FIG. 9).

When the above results are put into prospective, we can see that the GsMTx4 peptide or its derivatives of this invention is capable of relieving pain upon administration to animals treated with pain inducers. Thus, they can be useful analgesics in various proven pain models and medical situations.

The analgesic of this invention containing GsMTx4 or its derivatives as the active ingredient can be made into various orally and non-orally administered pharmaceutical formulations. Examples of orally administrable formulations include tablets and capsules. These oral formulations may further comprise the following ingredients besides the active one: diluents including lactose, dextrose, sucrose, mannitol, sorbitol, cellulose and glycine; lubricants including silica, talc, stearic acid and its salts, and polyethylene glycols. Tablets may further comprise binders including magnesium aluminum silicate, starch paste, gelatin, methyl cellulose, sodium carboxymethyl cellulose and polyvinylpyrrolidine. Tablets containing GsMTx4 of this invention may additionally include disintegrants such as starch, agar, alginic acid and its sodium salts; absorbents, colorings, flavorings and sweetners. These formulations can be manufactured by conventional processes such as mixing, granulation and coating. As for non-orally administrable formulations, injections are the representative formulation, preferably in the forms of aqueous solutions and suspension. These injection compositions can be sterilized and/or further contain additives and other therapeutically useful agents. Examples of these additives include preservatives, stabilizers, suspension concentrates or emulsifiers, salts for osmotic control and buffers. Such injections can be formulated via conventional methods.

The analgesic of the present invention can be administered according to therapeutic purposes by oral or non-oral routes such as intravenous, intramuscular and intraperitoneal routes. In the case of intraperitoneal administration, patients may take daily, in a single dose or more, 10 to 1000 µg or preferably, 40 µg per 150 g of their body weight of the inventive analgesic containing the GsMTx4 peptide or its derivatives as the active ingredient. Individual dose regimen may vary according to the patient's body weight, age, sex, health, diet, methods and times of administration, excretion, side reactions with other drugs and the severity of his condition.

### BRIEF SUMMARY OF THE INVENTION

The objective of the present invention is to provide a recombinant plasmid carrying a gene for a spider toxin peptide. Another objective of the invention is to provide a stable production method for said recombinant toxin peptide from yeast transformed with the plasmid mentioned above. It is still another objective of the invention to provide analgesic compositions containing said toxin peptide as the active ingredient.

To achieve these objectives, the present invention provides a recombinant plasmid for yeast expression that carries the gene for GsMTx4, a spider toxin peptide. The present invention also discloses a method for producing GsMTx4 in yeast transformed with said plasmid. Finally, the present invention provides analgesic compositions containing GsMTx4 or its derivatives as the active ingredient.

### BRIEF DESCRIPTION OF THE DRAWINGS

FIG. 1 provides cleavage maps for the vector pPICZαB and pGSMTX4, the latter carrying the gene for GsMTx4 in the backbone of pPICZαB.
FIG. 2 is an electrophoretogram of the purified GsMTx4 in a urea polyacrylamide gel.
FIG. 3 is a chromatogram of the purified GsMTx4 analyzed by HPLC.
FIG. 4 is a mass spectrum showing the molecular weight of GsMTx4 in MALDI-TOF mass spectrometry.
FIG. 5 is a graph demonstrating the analgesic effect of GsMTX4 in mechanical hyperalgesia heightened by a pain inducer.
FIG. 6 is a graph demonstrating the analgesic effect of GsMTX4 on mice against mechanical high-pressure stimuli. These mice were not treated with pain inducers.
FIG. 7 is a graph demonstrating the analgesic effect of GsMTX4 on mice against mechanical allodynia heightened by a pain inducer.
FIG. 8 is a graph demonstrating the analgesic effect of GsMTX4 on mice against mechanical low-pressure stimuli. These mice were not treated with pain inducers.
FIG. 9 is a graph demonstrating the analgesic effect of GsMTX4 on mice against mechanical stimuli heightened by a pain inducer.
FIG. 10 is a graph demonstrating the analgesic effect of GsMTX4 on mice against mechanical allodynia induced according to a neuropathic pain model.
FIG. 11 is a graph demonstrating the analgesic effect of GsMTX4 against heat stimuli. The mice were not treated with pain inducers.

### EXAMPLES

The invention is further described in detail by reference to the following experimental examples. These examples are provided for purposes of illustration only, and are not intended to be limiting unless otherwise specified.

### Example 1: Designing the base sequence for the gene encoding GsMTx4

Based on the known amino acid sequence of 34 residues, the present inventors designed the following DNA sequence encoding GsMTx4 without altering the amino acid residues in order to improve gene expression in the yeast *Pichia pastoris* by selecting codons preferred by this yeast (Graham, S. et al., Protein Expr. Purif. 26: pp96∼105, 2002).

10∼100 ng of genomic DNA isolated from the plastid transformants was used as the template for polymerase chain reaction (PCR). Using exTaq polymerase (Takara, Japan) with premix (Bioneer, Korea), the samples were pre-denatured for 5 minutes at 94°C and denatured for 1 minute at 94°C, followed by 1-minute primer annealing at 55°C. Then the samples were subject to chain extension for 1∼5 minutes at 72°C. This was repeated for 30 cycles and the samples were allowed for a final chain extension for 10 minutes at 72°C.

For a facile introduction of the above GsTMX4 gene into the expression vector pPICZαB (Invitrogen, USA) and extracellular secretion of the expressed GsTMx4 in high yields, the present inventors assembled the gene sequence as set forth in SEQ. ID NO 2 from 4 oligonucleotides (I), (II), (III) and (IV) which were synthesized using a DNA synthesizer (Applied Biosystems, USA). Oligonuceltides (I) and (II) as well as (III) and (IV), respectively, are complementary strands capable of forming duplex structures in suitable annealing conditions. Their base sequences are given below:

A secretion signal sequence of the α-factor containing an XhoI restriction site (**tc ga g**) connects to the 5' terminus of the sense strand of the structural gene for GsMTx4t (SEQ. ID NO 3), whereas a stop codon and an *Xal*I site (3'**-a gat c**-5') connect to the 5' terminus of the antisense strand (SEQ. ID NO 6) of the same structural gene.

### Example 2: Constructing an expression vector carrying the gene for GsMTx4

1-µL aliquots (100 pmol) of each of the four oligonucleotides (I), (II), (III) and (IV) were taken and mixed. This mixture was phosphorylated with T4 polynucleotide kinase (Boehringer Mannheim, Germany) and annealed to form a duplex. The duplex was nick-sealed by T4 DNA ligase (Boehringer Mannheim) and electrophoresed in an agarose gel. Fragment **1** of 129 base pairs encoding GsMTx4 was isolated.

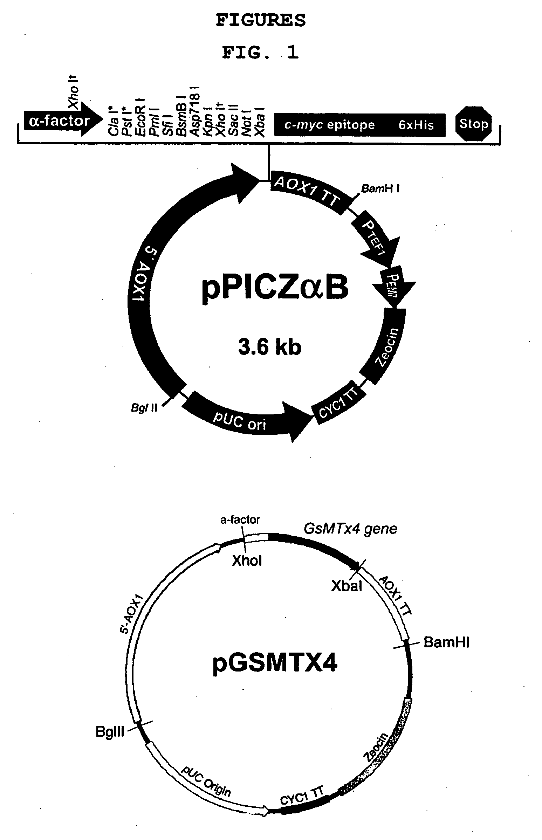

The pPICZαB was restricted with *Xho*I and *Xba*I to obtain fragment **2**, a 3300-bp fragment containing the alcohol oxidase 1 (AOX1) promoter, secretion signal from the α-factor, the AOX1 terminator and zeocin-resistance gene as a selection marker. Fragments **1** and **2** were ligated with T4 DNA ligase to construct the recombinant plasmid of this invention (FIG. 1).

This plasmid construct carries the gene for GsMTx4 downstream to the AOX1 promoter and the signal sequence as well as the AOX1 terminator and a selection marker. We named this plasmid pGSMTX4 and provided its structure in FIG. 1. As can be seen in the figure, this plasmid carries, in the following order, elements such as the AOX1 promoter::α-factor signal sequence::34-amino acid gene sequence for GsMTx4::AOX1 terminator and zeocin-resistance gene. This base sequence of genetic elements in this plasmid was determined by Sanger dideoxy sequencing (Sanger, F. et al., Proc. Natl. Acad. Sci. 74, pp5463∼5476, 1977).

DH5α *E. coli* cells were transformed with this plasmid and the transformants were deposited at the Korean Culture Center of Microorganisms (KCCM) under the accession number KCCM10652p on April 9^{th}, 2006.

### Example 3: Amplifying pGSMTX4 inside the E. coli transformants

The transformation of DH5α competent cells (Invitrogen, USA) with the plasmid pGSMTX4 of Example 2 was carried out as follows. pGSMTX4 was introduced into DH5α competent cells by electroporation. The competent cells were spread on low-salt Luria broth (LB) plates containing 25 µg/mL zeocin and were incubated at 37°C. Transformant colonies were picked and their plasmid DNA was checked with polymerase chain reaction (PCR) to look for genuine positive clones. From agarose gel electrophoresis results, we were able to confirm the presence of an approximately 129-bp DNA encoding GsMTx4 in a transformant colony. The cells from this colony were grown in zeocin-containing LB medium to isolate the plasmid DNA using a commercial midi-prep kit (Promega, USA).

### Example 4: Transformation of yeast cells and their selection

Methanol-assimilating *Pichia pastoris* yeast was transformed with pGSMTX4 for the producing of GsMTx4. The GS115 *Picha pastoris* cells (Invitrogen, USA) were cultured overnight in a 5 mL-YPD medium at 30°C. This starter culture was added to a fresh 25 mL-YPD medium and shaker-incubated at 30°C until an optical density (OD₆₀₀) of 1.0. The culture medium was then centrifuged for 5 minutes at 1,800 rpm and the resulting cell pellet was washed once with 25 mL of water, followed by washing with 25 mL of 1 M sorbitol for four times to obtain a cell suspension.

Meanwhile, the purified pGSMTx4 from Example 3 was linearized using a *Pme*I restriction digest and precipitated with ethanol. The precipitate was suspended at a concentration of 20 µg/10 µL. This plasmid suspension was added to the cell suspension mentioned above transformation of the yeast cells were carried out using the Gene pulser 2 electroporator (Bio-Rad, UK). The cell suspension was then spread on solid YPDS (yeast extract, peptone, dextrose and sorbitol) plates and the plates were incubated at 30°C for 2 to 3 days until colonies showed up.

To select positive clones with integrated GsMTx4 in the genome out of the colonies, genomic DNA was prepared and amplified with PCR for the presence of the gene for GsMTx4. Agarose gel electrophoresis confirmed a positive clone with GsMTx4.

### Example 5: Expressing GsMTx4

To express GsMTx4 in the transformed yeast cells, cells from the colony selected in Example 4 were inoculated in a minimal selective medium (0.67% yeast nitrogen base without amino acids, 2% glucose, 0.003% leucine, 0.002% histidine) with glucose as the carbon source. The cells were grown at 30°C for 3 days to finally yield about 100 colonies, which were in turn inoculated to a minimal selective medium containing methanol (0.67% yeast nitrogen base without amino acids, 0.5% methanol, 0.003% leucine, 0.002% histidine). A strain exhibiting robust growth was selected and named pGSMTX4/GS115.

pGSMTX4/GS115 was inoculated in a 100-mL YPD medium and shaker-incubated at 30°C for 48 hours. The culture medium was then removed by centrifugation and the cell pellet was resuspended and inoculated to a 1000 mL-BMMY medium (yeast extract, peptone, yeast nitrogen base, biotin, and methanol). The cells were shaker-incubated for 120 hours at 30°C with a methanol addition every 24 hours to keep the methanol level at 0.5% to induce the expression of GsMTx4 under the control of the AOX1 promoter.

A 1-mL aliquot was taken from the culture and spun down at 6,000 rpm for 5 minutes to obtain the supernatant and cell pellet. The supernatant was freeze-dried and then thawed in 10 µL of urea lysis buffer (50 mM Tris HCl, pH 6.8, 100 mM dithiothreitol, 2% SDS, 5% mercaptoethanol, 0.1% bromophenol blue). This was boiled for 2 minutes and the resulting solution was named the culture medium protein fraction. This fraction was electrophoresed for 20 minutes at 100 V and 20 mA where the gel was a urea polyacrylamide composite gel made of a 17% separating gel (pH 8.8, 10 cm X 5 cm X 1 mm) and a 5% stocking gel (pH 6.8, 10 cm X 1 cm X 1 mm). The electrophoresed gel was stained with Coomassie blue.

We were able to observe around 4 kDa, the band for the GsMTx4 peptide. This result indicates that GsMTx4 expressed inside the Pichia yeast was secreted to the extracellular medium by the ppL signal sequence from the recombinant vector. The yield confirmed was quite high (FIG. 2).

### Example 6: Purifying the GsMTx4 peptide

The 120-hour yeast culture from Example 5 was centrifuged for 30 minutes at 8,000 g to remove the cell pellet. The resulting supernatant was first filtered through a 3MM filter paper (Whatman, UK) to remove suspended material and then through a second filter paper with a pore size of 25 µL to remove fine particulate matter. Since the isoelectric point of GsMTx4 is about 9.1, the pH of the filtered supernatant was adjusted with glacial acetic acid to 4.0 for cation exchange chromatography. An SP-Sepharose^{™} cation exchange column (Pharmacia Biotech, USA) was equilibrated with 50 mM sodium acetate, pH 4 and then was loaded with the pH-adjusted supernatant. The column was washed with the same sodium acetate solution and the peptide was eluted with a salt gradient starting at 0 M to 2 M at pH 4.0. In the fraction eluting at 1.2~1.6 M salt, the peptide was co-eluted. This fraction was diluted 5-fold with the sodium acetate solution and was subject to another round of column chromatography using mono S cation exchange resin (Pharmacia Biotech). The resulting eluate was analyzed with HPLC using a C-14 column and a single peak was observed (FIG. 3). Absorbance measurement at 280 nm with a spectrophotometer gave a final yield of approximately 100 mg of GsMTx4 per liter.

### Example 7: Analysis of the GsMTx4 peptide

The GsMTx4 peptide purified in Example 6 was electrophoresed in a urea polyacrylamide gel and transferred to an Immobilion^{™} P membrane (Millipore, USA). It was then subject to Edman degradation for N-terminal sequence analysis with the Applied Biosystems 494 sequencing system (Perkin Elmer, USA). The first amino acid residue was glycine, but no further residues were detectable. This is attributable to the cysteine of the second residue forming a disulfide bond with another cysteine.

On the other hand, a MALDI-TOF mass spectrometry analysis with Voyager-DE^{™} STR workstation (Applied Biosystems, USA) gave a molecular weight of 4,094 Da, which is in exact agreement with the calculated value for GsMTx4.

### Example 8: Mechanical hyperalgesia - the Randall-Selitto test

The present inventors carried out the Randall-Selitto test in order to test whether GsMTx4 actually had an analgesic effect against mechanical stimuli inside a living body. A cone-shaped rod was placed at one of the hind paws of mice treated with GsMTx4 and force was applied. The threshold force at which mice exhibit a pain response was recorded. A pain response was characterized as either raising the hind paw, rolling up the tail, or making a shrill sound.

Two groups of 8 mice each were injected with either physiological saline solution or the pain inducer carrageenan (2% solution, 50 µL, intraplantar) at their hind paws. After the first injection, either GsMTx4 (40 µg/150 g body weight) or for the positive control group, morphine (1.5 mg/150 g) was intraperitoneally injected to these mice. The Randall-Selitto test was carried out 4 hours later. Mechanical stimuli were applied in an area in the paw between the thumb and index finger. Threshold weight data were represented as percentage values against the threshold weight for mice treated saline solution (negative control).

The test results show that for mice treated with carrageenan in their hind paws, the pain sensitivity threshold fell down 50% as inflammation developed in their paws; however, when these mice received an intraperitoneal injection of GsMTx4, their threshold value increased back substantially just as they did so for mice injected with morphine, a central analgesic. This analgesic effect of GsMTx4 occurred 30 minutes after its injection and lasted for approximately 4 hours (FIG. 5).

The analgesic effect of GsMTx4 against mechanical high pressure was also evident in the group of mice that did not receive carrageenan when this group was compared to those that received intraplantar saline (50 µL) and intraperitoneal morphine injections (FIG. 6).

We were thus able to conclude from these results, a strong activity of the GsMTx4 peptide in suppressing pain from mechanical stimuli.

### Example 9: Mechanical allodynia - the von Frey test

The present inventors conducted the von Frey test in order to see whether GsMTx4 of this invention has an analgesic effect against rubbing or other weak mechanical stimuli inside a living body. When a pain inducer is administered only to one of the hind paws of a mouse, the affected paw becomes sensitive and feels pain from even small mechanical stimuli. The von Frey test involves measuring the weight difference at the pain threshold between the two hind paws as one varies the width of the von Frey hair involved in giving the stimuli (http://www.2biol.com/2biol analgesia.htm; Pitcher, G. M. et al., Journal of Neuroscience Methods 87(2):185∼193, 1999).

Two groups of 8 mice each received a 50-µL intraplantar injection of either physiological saline solution or 2% carrageenan at only one of their hind paws. After the first injection, either GsMTx4 (40 µg/150 g body weight) or for the positive control group, morphine (10 mg/150 g) was intraperitoneally injected to these mice. These mice were rubbed with von Frey hairs a half to 4 hours later.

Mechanical stimuli were applied in an area in the paw between the thumb and index finger. Threshold weight data were represented as percentages against the threshold weight for mice treated saline solution (negative control).

There was no difference in the endurance against hair force among mice treated with saline solution alone and those mice that did not receive saline. Mice injected with 2% carrageenan in one of their limbs showed a large drop in their pain threshold for hair force. When GsMTx4 or morphine was injected to the carrageenan-treated mice, both injections were observed to reduce hypersensitivity. This indicates that as in the case of morphine, GsMTx4 has an analgesic effect (FIG. 7).

The analgesic effect of GsMTx4 against mechanical low pressure was also observed, albeit weakly, in the group of mice that did not receive carrageenan when this group was compared to those that received intraplantar saline (50 µL) and intraperitoneal morphine injections (FIG. 8).

### Example 10: The weight-bearing test

In order to look into whether the GsTx4 peptide of this invention has analgesic effect against mechanical stimuli, the present inventors carried out the weight-bearing test with mice.

The weight-bearing test was carried out according to the method of Min *et al*. (Min, S. et al., Neurosci. Lett. 308:95∼98, 2001). More specifically, the principles behind this test are as follows. When a mouse receives an injection of pain inducers in only one of the hind paws, it puts more weight than usual in the other hind paw. This difference in the weight balance serves as a measure of the mouse's pain. In the present example, such weight difference per unit body weight of the mouse was reported for comparison.

Two groups of 8 mice each received an intraplantar injection of either 2% carrageenan (50 µL) or for the negative control group, physiological saline solution (100 µL) at only one of their hind paws. To the carrageenan-treated mice, either GsMTx4 (40 µg/150 g body weight) or for the positive control group, morphine (10 mg/150 g) was intraperitoneally and their weight balances were monitored 1 to 6 hours later.

For mice treated with saline solution only, there was no weight difference between their hind limbs, but mice treated with 2% carrageenan showed a substantial increase in the weight difference between them. When GsMTx4 or morphine was injected to the carrageenan-treated mice, the substantial weight difference induced by the injection of carrageenan reverted back to a low value (FIG. 9).

These results confirmed the analgesic activity of the GsMTx4 similar to that of morphine.

### Example 11: A test for pain induced by mechanical stimuli under a neuropathic pain model

In order to test whether the analgesic effect of GsMTx4 against mechanical stimuli extends to neuropathic pain inside a living body, the present inventors induced neuropathic pain in mice and applied mechanical stimuli to examine the neuropathic pain responses of the affected mice.

Neuropathic pain was induced to mice according to the method reported by Lee and others (Lee, B. et al., Neuroreport 11:657∼661, 2000). Mice were put under anesthesia and their skin was excised in the thighs to expose the tibial, sural and common peroneal nerves. Out of these, the tibial and sural nerves were bound together and cut off. Mice were then allowed to recuperate for 6 days after the operation and the monitoring for the presence of neuropathic pain began 1 week after the operation. The analgesic effect of GsMTx4 on those mice suffering from neuropathic pain was tested by the von Frey test as in Example 9.

Mice with neuropathic pains were divided into two groups of 9. To the first group, GsMTx (50 µg/200 g body weight) was injected and to the second group, physiological saline solution for control. 4 hours after the injection of GsMTx4, mechanical stimuli were applied to these mice with von Frey filaments. The width of the von Frey filament that evoked a nociceptic (flexor) reflex was recorded before and after the surgery, and the data was expressed as the percentage of the latter width in relation to the former.

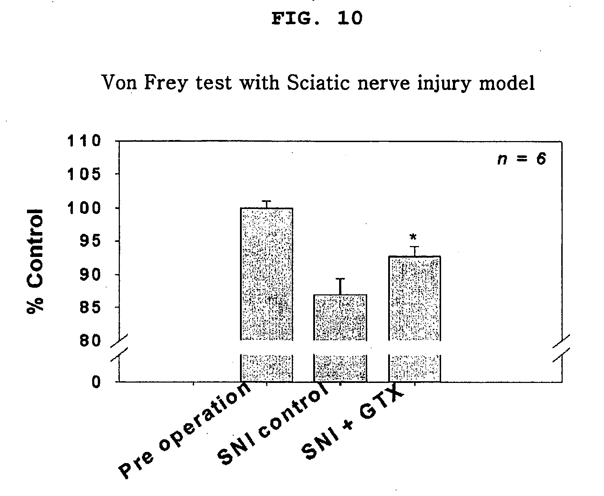

The results showed an elevation of the reflex threshold against mechanical stimuli in those mice that received intravenous injections of GsMTx4 over that of the control group. Thus, the present inventors concluded that GsMTx4 was capable of suppressing pain from mechanical stimuli applied at the hind limbs of mice suffering an induced neuropathic pain (FIG. 10).

### Example 12: Temperature stimuli - The tail flick test

The present inventors looked into GsMTx4 for its possible analgesic activity on pain from temperature stimuli. Mice were irradiated with infrared in their tails and monitored for their endurance against heat (tail flick test). The analgesic effect of GsMTx4 was examined by comparing data from a group of mice suffering from induced pain with those from a non-induced group (Pitcher G. M. et al., Journal of Neuroscience Methods, 87(2):185∼193, 1999).

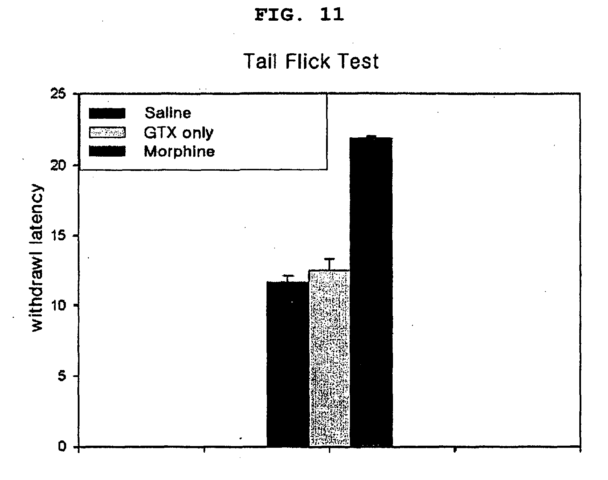

Three groups of 8 mice each respectively received an intraperitoneal injection of either physiological saline solution (100 µL) as the negative control, or morphine (10 mg/150 g body weight) as the positive control, or GsMTx4 (40 µg/150 g). They were then subject to the tail flick test 30 to 4 hours later. When compared to morphine, the GsMTx4 peptide had either negligible or no analgesic activity against temperature stimuli (FIG. 11).

### INDUSTRIAL APPLICABILITY

The GsMTx4 peptide of this invention is a specific inhibitor of great utility for blocking mechanosensitive ion channels. Through expression of GsMTx4 in yeast cells transformed with an inventive recombinant plasmid construct, the present invention also provides a more stable production method for manufacturing GsMTx4 than its natural route from a tarantula toxin. Analgesic compositions containing the inventive GsMTx4 or its derivatives may find use not only in experiments in various verified pain models and medical application but also in the cure of such diseases as heart failure.

### LIST OF SEQUENCES

SEQ. ID NO 1 is the amino acid sequence for the GsMTx4 peptide.

SEQ. ID NO 2 is the full-length nucleotide sequence for GsMTx4.

SEQ. ID NO 3 is the base sequence for oligonucleotide (I) used in the assembly of the full-length GsMTx4 nucleotide sequence.

SEQ. ID NO 4 is the base sequence for oligonucleotide (II) used in the assembly of the full-length GsMTx4 nucleotide sequence.

SEQ. ID NO 5 is the base sequence for oligonucleotide (III) used in the assembly of the full-length GsMTx4 nucleotide sequence.

SEQ. ID NO 6 is the base sequence for oligonucleotide (IV) used in the assembly of the full-length GsMTx4 nucleotide sequence.

SEQ. ID NO 7 is the base sequence of the secretion signal sequence for the alpha factor.

## Claims

1. A GsMtx4 peptide having the amino acid sequence of SEQ ID NO 1 for use as an analgesic.

2. The GsMTx4 peptide according to claim 1, wherein said analgesic relieves pain by blocking ion channels which are gated by external stimuli to suppress volume-activated current.

3. The GsMTx4 peptide according to claim 2, wherein the external stimuli are mechanical.

4. The GsMTx4 peptide according to claim 2, wherein the pain is either the pain inflicted by the external stimuli or inflammatory pain.

5. The GsMTx4 peptide according to claim 2, wherein said ion channels include stretch-activated channels (SACs) and mechanosensitive channels (MSC).

## Patentansprüche

1. GsMtx4 Peptid, das die Aminosäuresequenz SEQ ID NO 1 aufweist, zur Verwendung als ein Analgetikum.

2. GsMTx4 Peptid nach Anspruch 1, wobei das genannte Analgetikum Schmerz lindert durch Blockieren von Ionenkanälen, die durch externe Reize gesteuert werden, um volumenaktivierten Strom zu unterdrücken.

3. GsMTx4 Peptid nach Anspruch 2, wobei die externen Reize mechanisch sind.

4. GsMTx4 Peptid nach Anspruch 2, wobei der Schmerz entweder Schmerz ist, der durch externe Reize zugeführt worden ist, oder Entzündungsschmerz ist.

5. GsMTx4 Peptid nach Anspruch 2, wobei die genannten Ionenkanäle dehnungsaktivierte Kanäle (SACs) und mechanosensitive Kanäle (MSC) beinhalten.

## Revendications

1. Peptide GsMtx4 ayant la séquence d'acides aminés de SEQ ID NO 1 pour une utilisation comme analgésique.

2. Peptide GsMTx4 selon la revendication 1, dans lequel ledit analgésique soulage une douleur en bloquant les canaux ioniques qui sont activés par des stimuli externes pour supprimer un courant activé par le volume.

3. Peptide GsMTx4 selon la revendication 2, dans lequel les stimuli externes sont mécaniques.

4. Peptide GsMTx4 selon la revendication 2, dans lequel la douleur est soit la douleur infligée par les stimuli externes, soit une douleur inflammatoire.

5. Peptide GsMTx4 selon la revendication 2, dans lequel lesdits canaux ioniques incluent des canaux activés par l'étirement (SAC) et les canaux mécano-sensibles (MSC).


### SEQUENCE LISTING

<110> Seoul National University Industrial Foundation
<120> recombinant yeast expression plasmid having GsMTx4 gene and method for mass-producing GsMTx4, Grammostola spatulata toxin peptide using the same
<160> 7
<170> KopatentIn 1.71
<210> 1
   <211> 34
   <212> PRT
   <213> GsMTx-4 peptide
<400> 1
   Gly Cys Leu Glu Phe Trp Trp Lys Cys Asn Pro Asn Asp Asp Lys Cys
     1               5                  10                  15
   Cys Arg Pro Lys Leu Lys Cys Ser Lys Leu Phe Lys Leu Cys Asn Phe
                20                  25                  30
   Ser Phe
<210> 2
   <211> 102
   <212> DNA
   <213> Artificial Sequence
<220>
   <223> GsMTx-4 DNA sequence
<400> 2
   ggttgtttgg agttctggtg gaagtgcaac ccaaacgacg acaagtgttg cagaccaaag      60
   ttgaagtgtt ccaagttgtt caagttgtgc aacttctctt tt                         102
<210> 3
   <211> 68
   <212> DNA
   <213> Artificial Sequence
<220>
   <223> sense(1)
<400> 3
   tcgagaaaag agaggctgaa gctggttgtt tggagttctg gtggaagtgc aacccaaacg 60
   acgacaag 68
<210> 4
   <211> 64
   <212> DNA
   <213> Artificial Sequence
<220>
   <223> antisense(2)
<400> 4
   cttgtcgtcg tttgggttgc acttccacca gaactccaaa caaccagctt cagcctctct 60
   tttc 64
<210> 5
   <211> 61
   <212> DNA
   <213> Artificial Sequence
<220>
   <223> sense(3)
<400> 5
   tgttgcagac caaagttgaa gtgttccaag ttgttcaagt tgtgcaactt ctctttttga 60
   t 61
<210> 6
   <211> 65
   <212> DNA
   <213> Artificial Sequence
<220>
   <223> antisense(4)
<400> 6
   ctgaatcaaa aagagaagtt gcacaacttg aacaacttgg aacacttcaa ctttggtctg 60
   caaca 65
<210> 7
   <211> 267
   <212> DNA
   <213> alpha-factor preproleader sequence
<400> 7
   atgagatttc cttcaatttt tactgctgtt ttattcgcag catcctccgc attagctgct 60
   ccagtcaaca ctacaacaga agatgaaacg gcacaaattc cggctgaagc tgtcatcggt 120
   tactcagatt tagaagggga tttcgatgtt gctgttttgc cattttccaa cagcacaaat 180
   aacgggttat tgtttataaa tactactatt gccagcattg ctgctaaaga agaaggggta 240
   tctctcgaga aaagagaggc tgaagct 267